# FASCICULE DE BREVET EUROPEEN

(11) **EP 2 611 903 B1**
(45) Date de publication et mention de la délivrance du brevet: **05.10.2016**
(21) Numéro de dépôt: 11764808.9
(22) Date de dépôt: 30.08.2011
(51) Int. Cl.: C12N 3/00, C12Q 1/04

(54) **UTILISATION D'UN ACTIVATEUR DE BETA-GLUCOSIDASE POUR LA DÉTECTION ET/OU L'IDENTIFICATION DE C.DIFFICILE**
VERWENDUNG EINES BETA-GLUCOSIDASEAKTIVATORS FÜR DEN NACHWEIS UND/ODER DIE IDENTIFIZIERUNG VON C.DIFFICILE
USE OF A BETA-GLUCOSIDASE ACTIVATOR FOR DETECTING AND/OR IDENTIFYING C. DIFFICILE

(30) Priorité: 01.09.2010 FR 1056928
(43) Date de publication de la demande: 10.07.2013
(73) Titulaire: Biomérieux, 69280 Marcy L'Etoile (FR)
(72) Inventeur: CELLIER, Marie, 38390 Montalieu Vercieu (FR); HALIMI, Diane, 01700 Saint-Maurice-de-Beynost (FR); ORENGA, Sylvain, 01160 Neuville sur Ain (FR)
(86) Numéro de dépôt international: PCT/FR2011/051982
(87) Numéro de publication internationale: WO 2012/028816

(56) Documents cités:
- WO-A1-98/45469
- FR-A1- 2 912 423
- FR-A1- 2 926 563
- US-A1- 2007 004 021
- HILDEBRAND D C ET AL: "beta-Glucosidase Activity in Phytopathogenic Bacteria.", APPLIED MICROBIOLOGY NOV 1964 LNKD- PUBMED:16349648, vol. 12, no. 6, novembre 1964 (1964-11), pages 487-491, XP002621316, ISSN: 0003-6919 cité dans la demande
- GILBRETH STEFANIE EVANS ET AL: "Catabolite repression and virulence gene expression in Listeria monocytogenes.", CURRENT MICROBIOLOGY AUG 2004 LNKD- PUBMED:15297913, vol. 49, no. 2, août 2004 (2004-08), pages 95-98, XP002621317, ISSN: 0343-8651
- WILSON K H ET AL: "Use of sodium taurocholate to enhance spore recovery on a medium selective for Clostridium difficile.", JOURNAL OF CLINICAL MICROBIOLOGY MAR 1982 LNKD- PUBMED:7076817, vol. 15, no. 3, mars 1982 (1982-03), pages 443-446, XP002621318, ISSN: 0095-1137
- J BACTERIOL ET AL: 'Neurospora crassa in -Glucosidases [beta] Induction of', [en ligne] 01 Janvier 1973, page 295, XP055115569 Extrait de l'Internet: <URL:http://jb.asm.org/content/116/1/295.fu ll.pdf> [extrait le 2014-04-28]

## Description

La présente invention concerne un procédé de détection et/ou d'identification des *Clostridium difficile.* Elle concerne également un milieu réactionnel spécifique de *Clostridium difficile* comprenant un activateur de beta-glucosidase.

*Clostridium difficile (C. difficile)* est un bacille à Gram positif, anaérobie strict, et formant des spores. Il est à l'origine de pathologies intestinales de gravité variable : diarrhées moyennes ou fulminantes, colites pseudomembraneuses ou megacôlon toxique pouvant lui-même être à l'origine d'un sepsis. *Clostridium difficile* est transmis par ingestion orale de spores qui résistent à l'acidité de l'estomac et germent dans l'intestin. Un déséquilibre de la flore commensale du côlon permet la prolifération de C. *difficile* qui produit des toxines à l'origine des colites. Dans de nombreux cas, le déséquilibre de la flore commensale est consécutif à une prise d'antibiotiques. *C*. *difficile* peut être considéré comme un microorganisme inoffensif de l'environnement, qui devient pathogène opportuniste dans des conditions particulières. Le portage digestif asymptomatique de *C*. *difficile* est estimé à 3% dans la population adulte mais il peut atteindre 15 à 25% des sujets après un traitement antibiotique ou un séjour dans une unité à forte endémicité. Un taux de portage élevé est habituellement observé, de 20 à 40% chez des patients hospitalisés jusqu'à 50-70% chez les jeunes enfants. La possibilité d'un diagnostic précoce et rapide est un élément clé pour la prévention des complications sévères, et pour une prise en charge clinique efficace.

Le diagnostic repose sur la recherche des toxines et sur la culture.

La mise en évidence des toxines directement à partir des selles est un excellent marqueur de la présence d'une souche toxinogène de *C*. *difficile.* La méthode de référence consiste à rechercher un effet cytopathogène (CTA pour cytotoxic activity selon l'appellation anglo-saxonne) par culture cellulaire. Cette méthode est très sensible mais n'est pas standardisée et nécessite des techniciens spécialisés et un délai de réponse de plusieurs jours. Des tests immunoenzymatiques de type ELISA ont également été développés, qui détectent soit la toxine A seule, soit les toxines A et B. Le temps d'obtention d'un résultat est réduit. Il est toutefois recommandé d'associer les techniques immunologiques utilisées pour la détection des toxines à la culture bactérienne pour isoler les souches de *C*. *difficile.* Cette culture peut être mise en oeuvre sur une gélose *C*. *difficile* commercialisée par la Demanderesse, les colonies de *C*. *difficile* étant ensuite identifiées par des méthodes biochimiques telles que la galerie API® 20 A ou la galerie rapid ID 32 A. Toutefois, l'identification par galerie nécessite d'avoir une culture pure de la bactérie à identifier, sur un milieu de croissance adapté afin d'obtenir suffisamment de biomasse (3 à 4 Mc Farland) ce qui prend, au mieux, 48h (culture d'isolement de 24 à 72 heures à partir du prélèvement donnant uniquement la détection présomptive puis culture de 24 à 72 heures pour générer suffisamment de biomasse). La lecture de la galerie s'effectue ensuite après 4h d'incubation pour la rapid ID 32 A et 24 à 48h pour la galerie 20A.

Enfin, le diagnostic peut être réalisé par des techniques de biologie moléculaire. Ces techniques ne sont toutefois pas utilisées en routine à ce jour.
Par ses travaux dans le but d'améliorer l'identification directe de *C*. *difficile,* dans une culture bactérienne, la Demanderesse a mis en évidence que l'utilisation de substrat(s) enzymatique(s) de beta-glucosidase permet une identification aisée et rapide de *C*. *difficile.* Ladite utilisation de substrat de beta-glucosidase est décrite dans la demande WO 2009/092982 comme diminuant le temps d'obtention d'un résultat d'identification de *C*. *difficile.*

L'identification précoce et rapide de *C*. *difficile* susceptible d'être présent dans un échantillon reste un objectif prioritaire et la Demanderesse a poursuivi ses travaux en cherchant plus particulièrement à accélérer la révélation de l'activité enzymatique. Elle a ainsi mis en évidence de façon inattendue qu'un milieu réactionnel comprenant un substrat de beta-glucosidase et un activateur de beta-glucosidase de formule générale Ar-beta-D-glucopyranoside, où Ar- correspond à un noyau aromatique ou à plusieurs noyaux aromatiques accolés, permet une identification rapide de *C*. *difficile* dans un échantillon, autrement dit qu'un tel milieu permet une détection plus précoce de l'activité enzymatique d'au moins certaines souches de *C*. *difficile.* L'utilisation d'un composé Ar-beta-D-glucoside permet une activation de la beta-glucosidase et l'observation d'une coloration significativement plus intense des colonies de *C*. *difficile.* Avantageusement, les composés Ar-beta-D-glucoside ont un pouvoir activateur de la beta-glucosidase de *C*. *difficile* très supérieur à celui d'autres dérivés beta-D-glucoside tels le cellobiose (4-O-beta-D-glucopyranosyl-D-glucose) ou le Méthyl-beta-D-glucose.

Il est connu que les composés de formule générale Ar-beta-D-glucoside, où Ar- représente au moins un noyau aromatique, homo- ou hétéro-cyclique, peuvent être utilisés comme substrats de beta-glucosidase. Cette utilisation est rapportée dans la demande WO 2009/092982 *(supra).* De même, Hildebrand et Schroth rapportent que l'arbutine, ou hydroquinone beta-D-glucopyranoside, est utilisée comme source de carbone, à une concentration de 0.5% poids/volume, autrement dit de 5 g/l, en combinaison avec du glucose, dans un milieu réactionnel. L'hydrolyse de l'arbutine en hydroquinone, mesurée par des méthodes chromatographiques, est un marqueur de l'activité beta-glucosidase de *Pseudomonas syringae* (Hildebrand et Schroth, Applied Microbiology, 1964 ; 12 (6) : 487-491). Ceci dissuade l'homme du métier d'utiliser un composé Ar-beta-D-glucoside comme activateur de beta-glucosidase dans un milieu réactionnel pour la détection ou l'identification de *C*. *difficile* puisqu'il se trouverait en compétition avec le substrat destiné à révéler l'activité enzymatique du microorganisme cible.

Enfin, l'art antérieur rapporte que l'arbutine administrée par voie orale est excrétée et métabolisée dans les urines et peut, à ce titre, être utilisée pour ses propriétés diurétiques et antibactériennes (Schindler et al., 2002 ; J . Clin. Pharmacol. ; 42 (8) : 920-927 ; Siegers et al., 2003, Phytomedicine ; 10 (Suppl 4) :58-60). Une telle utilisation dissuade également d'utiliser l'arbutine dans un milieu réactionnel pour détecter une bactérie.

Avant une description détaillée de l'invention, les définitions suivantes sont données pour permettre de mieux comprendre l'invention. Elles ne sont nullement limitatives.

Par milieu réactionnel, on entend un milieu comprenant tous les éléments nécessaires à l'expression d'un métabolisme et/ou à la croissance de microorganismes. Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine, de l'agarose ou d'autres gélifiants naturels ou artificiels. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press). Le milieu réactionnel selon l'invention doit permettre la croissance des *C*. *difficile.*

Le milieu réactionnel peut comprendre un ou plusieurs éléments en combinaison, tels que des acides aminés, des peptones, des hydrates de carbone, des nucléotides, des minéraux, des vitamines ... Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant, un ou plusieurs indicateurs métaboliques, un ou plusieurs régulateurs métaboliques...

Le milieu réactionnel peut être un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture. L'homme du métier peut également utiliser une bi-boite, permettant de comparer aisément deux milieux, comprenant différents substrats ou différents mélanges sélectifs, sur lesquels on aura déposé un même échantillon biologique.

Le milieu réactionnel peut comprendre un ou plusieurs activateurs de croissance des souches de *C*. *difficile.* Par activateur de croissance, on entend un composé ou un groupe de composés qui stimule la croissance de microorganismes. On peut citer notamment le sang, sérum, jaune d'oeufs. Sans être limitatif, une concentration comprise entre 0,1 et 10 % est particulièrement adaptée à la présente invention.

Le milieu réactionnel peut comprendre un ou plusieurs agents réducteurs. Par agent réducteur, on entend un composé ou un groupe de composés qui facilite la croissance des germes anaérobies en neutralisant l'oxygène dissout présent dans le milieu. On peut citer notamment la Cystéine, le Pyruvate, l'Oxyrase, le Sulfite de sodium, la Dithionite, l'Histidine, le Sulfide ferreux. Sans être limitatif, une concentration comprise entre 0,05 et 50 g/1, préférentiellement entre 0,1 et 2 g/l est particulièrement adaptée à la présente invention.

Le milieu réactionnel peut comprendre un ou plusieurs inducteurs de la germination des spores de *C*. *difficile.* Par inducteur de la germination des spores, on entend un composé ou un groupe de composé qui favorise le passage de l'état de spore à l'état végétatif des *C*. *difficile.* On peut citer notamment le Taurocholate de Sodium.

Sans être limitatif, une concentration comprise entre 0,1 et 10 g/1, préférentiellement entre 1 et 5 g/l est particulièrement adaptée à la présente invention.

Le milieu réactionnel peut comprendre un ou plusieurs agents sélectifs. Par agent sélectif, on entend tout composé susceptible d'empêcher ou de ralentir la croissance d'un microorganisme autre que le microorganisme cible. Sans être limitatif, une concentration comprise entre 5 mg/l et 5 g/l est particulièrement adaptée à la présente invention.

On peut citer comme agent sélectif, les antibiotiques, les antifongiques. Par antibiotique, on entend tout composé susceptible d'empêcher ou de ralentir la croissance d'une bactérie. Ils appartiennent notamment aux groupes des céphalosporines, des aminosides, des polypeptides, des sulfamides, des quinolones. A titre indicatif, on peut citer notamment les antibiotiques cefotaxime, ceftazidime, cefoxitine, ceftriaxone, cefpodoxime, aztréonam, gentamicine, Triméthoprime, tobramycine, moxalactam, fosfomycine, D-cylcosérine, Polymixine, Colistine, les quinolones telles que l'acide nalidixique.

Par antifongique, on entend tout composé susceptible d'empêcher ou de ralentir la croissance d'une levure ou d'une moisissure. A titre indicatif, on peut citer notamment l'amphotéricine B, le fluconazole, l'itraconazole, le voriconazole, la cycloheximide.

D'une manière générale, le milieu réactionnel peut en sus contenir un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou chromogène. Pour une détection indirecte, le milieu réactionnel selon l'invention peut comporter en sus un indicateur de pH, sensible à la variation de pH induite par la consommation du substrat et révélant la croissance des microorganismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'hydroxycoumarine ou les dérivés de la résorufine.

Par substrat de beta-glucosidase, apte à l'identification de *C*. *difficile,* on entend un substrat qui induit, dans des conditions de croissance adaptées, la coloration de *C*. *difficile.* On entend notamment le 2 Hydroxyphényl-beta glucoside (Catéchol-beta glucoside); Magenta-beta glucoside (5 Bromo-6 chloro-3 indoxyl-beta glucoside); DHF-beta glucoside (Dihydroxyflavone-beta glucoside) ; l'Esculine (Esculétine-beta glucoside) ; CHE-beta glucoside (3,4 Cyclohexénoesculétine-beta glucoside) ; 8 Hydroxiquinoline-beta glucoside ; X-beta glucoside (5 Bromo-4 chloro-3 indoxyl-beta glucoside) ; Rose-beta glucoside (6 Chloro-3 indoxyl-beta glucoside) ; 6 Bromo-3 indoxyl-beta glucoside ; Blue-beta glucoside (5 Bromo-3 indoxyl-b glucoside) ; 6 Fluoro-3 indoxyl-beta glucoside ; Alizarine-beta glucoside ; (P) Nitrophényl-beta glucoside ; 4 Méthylumbelliferyl-beta glucoside, Naphtholbenzein-beta glucoside, Indoxyl-N méthyl-beta glucoside, 5 Bromo-4 chloro-3 indoxyl-N méthyl-beta glucoside, le Naphtyl-beta glucoside ; l'Aminophényl-beta glucoside ; le Dichloroaminophényl-beta glucoside, les Aldol™-beta-glucoside (BIOSYNTH, Lukas M. Wick, Alexander Bayer, Christophe Weymuth, Günter Schabert, Vanessa Pfister, Aysel Aslan, Urs P. Spitz, « Novel Chromogenic Enzyme Substrates », ASM General Meeting, 2009, Philadelphia, I-091/285).

Le milieu réactionnel peut comprendre en outre un activateur enzymatique. Par activateur enzymatique, on entend un composé qui sert à activer le processus de digestion enzymatique, autrement dit à réduire le temps d'incubation pour révéler l'activité enzymatique et/ou à accroitre ladite activité enzymatique.

Les activateurs de beta-glucosidase peuvent avoir pour formule générale Ar-beta-D-glucoside, où Ar- représente un noyau aromatique. Par noyau aromatique, on entend un composé insaturé, c'est-à-dire présentant des doubles liaisons et comportant un ou plusieurs cycles accolés, homo- ou hétéro-atomiques, chaque cycle ayant de 5 à 7 sommets. De manière non limitative, on citera l'arbutine (hydroquinone-beta-D-glucoside), le 4-methylumbelliferyl-beta-glucoside, le 4 aminophenyl-beta-glucoside, la salicine (2-(hydroxymethyl)-phenyl-beta-D-glucoside), l'esculine (6,7dihydroxy-coumarine-beta-glucoside). Certains de ces composés sont des substrats de la beta-glucosidase. Au titre de l'invention, ils sont considérés comme des activateurs car ils permettent de réduire le temps d'incubation nécessaire à la détection de l'hydrolyse du composé utilisé comme substrat, ou ils permettent d'accroître cette hydrolyse. Ainsi, de façon surprenante, la combinaison d'un substrat enzymatique et d'un tel activateur permet d'avoir une détection plus précoce et/ou plus intense de l'activité enzymatique que lorsque ces composés sont utilisés séparément.

Le pouvoir activateur d'un composé peut notamment être mis en évidence pour certaines souches de *C. difficile* ayant une activité beta-glucosidase tardive et/ou faible, si la combinaison dudit composé et du substrat permet une détection plus précoce et/ou plus intense que si le substrat ou l'activateur était utilisé seul à une molarité au moins équivalente à la somme des molarités du substrat et de l'activateur en combinaison.

Par échantillon biologique, on entend une petite partie ou petite quantité isolée d'une entité pour l'analyse. Ce peut être un échantillon clinique, issu d'un prélèvement de liquide biologique ou un échantillon alimentaire, issu de tout type d'aliment. Cet échantillon peut être ainsi liquide ou solide. On peut citer d'une manière non limitative, un échantillon clinique de sang total, de sérum, de plasma, d'urines, de fécès, de prélèvements de nez, de gorges, de peau, de plaies, de liquide céphalorachidien, un échantillon alimentaire d'eau, de boissons tels que le lait, un jus de fruits, de yaourt, de viande, d'oeufs, de légumes, de mayonnaise, de fromage ; de poisson..., un échantillon alimentaire issu d'une alimentation destinée aux animaux, tel que notamment un échantillon issu de farines animales. Ce prélèvement peut être utilisé tel quel ou, préalablement à l'analyse, subir une préparation de type enrichissement, extraction, concentration, purification, selon des méthodes connues de l'homme du métier.

Après mise en contact avec l'échantillon, le milieu réactionnel est incubé à une température appropriée, généralement comprise entre 20 et 50°C, préférentiellement entre 30 et 40°C. De façon préférentielle, l'incubation est effectuée en anaérobiose, autrement dit en l'absence d'oxygène, selon des techniques connues de l'homme du métier.

A ce titre, l'invention concerne un milieu réactionnel comprenant au moins un substrat de beta-glucosidase, et un composé de formule générale Ar-beta-D-glucoside, où Ar- désigne un composé aromatique, différent dudit substrat et choisi parmi l'arbutine ou hydroquinone-beta-D-glucopyranoside, le 4-methylumbelliferyl-beta-glucoside, le 4-aminophenyl-beta-glucoside, la salicine ou 2-(hydroxymethyl)phenyl-beta-D-glucoside, à une concentration inférieure à 5 g/l. Préférentiellement, le composé Ar-beta-D-glucoside est à une concentration comprise entre 0,3 et 1,5 g/l.

De façon préférentielle, le composé Ar-beta-D-glucoside est l'arbutine.

Selon un mode préféré de réalisation de l'invention, le substrat de beta-glucosidase est choisi parmi l'Alizarine-beta-glucoside, le Magenta-beta-glucoside (5-Bromo-6-chloro-3-indoxyl-beta-glucoside), le DHF-beta-glucoside, le 3HF-beta-glucoside, le CHE-beta-glucoside (3,4-Cyclohexenoesculetine-beta-glucoside) et les ALDOL™-beta-glucoside de la société BIOSYNTH (Wick et al. 2009. ASM General meeting - Philadelphia (PA, USA)). Ledit substrat de beta-glucosidase est à une concentration comprise entre 25 et 1 000 mg/l, préférentiellement entre 50 et 400 mg/l.

Selon l'invention, l'activateur enzymatique est un substrat enzymatique dont le produit d'hydrolyse est différent du produit de l'hydrolyse du substrat enzymatique.

Selon un mode préféré de réalisation de l'invention, l'activateur enzymatique est un substrat enzymatique dont le produit n'est pas détectable à l'oeil nu dans le milieu réactionnel, alors que le produit de l'hydrolyse du substrat enzymatique l'est.

Selon un autre mode préféré de réalisation de l'invention, le milieu réactionnel comprend en outre un activateur de la germination des spores de *C. difficile,* ledit activateur étant préférentiellement le taurocholate de sodium, à une concentration comprise entre 0,1 et 10,0 g/l, préférentiellement comprise entre 1,0 et 5,0 g/l.

L'invention concerne également un procédé de détection et/ou d'identification de *C*. *difficile* comprenant les étapes consistant à :
a) mettre en contact un échantillon susceptible de contenir *C*. *difficile* avec un milieu réactionnel comprenant au moins un substrat de beta-glucosidase et un composé de formule générale Ar-beta-D-glucoside, différent dudit substrat, où Ar- désigne un composé aromatique, choisi parmi l'arbutine ou hydroquinone-beta-D-glucopyranoside, le 4-methylumbelliferyl-beta-glucoside, le 4-aminophenyl-beta-glucoside, la salicine ou 2-(hydroxymethyl)phenyl-beta-D-glucoside, à une concentration inférieure à 5 g/l;
b) incuber, et
c) détecter l'hydrolyse du substrat de beta-glucosidase, indiquant la présence de *C*. *difficile.*

Selon un mode préférentiel de réalisation, le procédé selon l'invention met en oeuvre à l'étape a) un milieu tel que défini *supra.*

Selon un autre mode préférentiel de réalisation, l'incubation mise en oeuvre à l'étape b) du procédé selon l'invention est réalisée en anaérobiose.

Les exemples ci-dessous sont donnés à titre explicatif et n'ont aucun caractère limitatif. Ils permettront de mieux comprendre l'invention.

### Exemple 1 : mise en évidence de l'effet activateur de l'arbutine sur un substrat de beta-glucosidase.

### 1. MILIEU ET MICROORGANISMES

Huit souches de *Clostridium difficile,* de la collection de la Demanderesse, ont été testées sur des milieux comprenant de l'arbutine à différentes concentrations en présence ou non de CHE-beta-glucoside. La lecture des boites est ensuite effectuée à 24h. Les milieux se composent comme suit :
Base ChromID® *C*. *difficile* commune à tous les milieux :

| Composés | Conc. en g/l |
|---|---|
| Peptone, extraits tissulaires et cellulaires | 12.5 |
| Chlorure de sodium | 6 |
| Sulfate de Magnésium | 0,3 |
| L-arginine | 1 |
| Bisulfite de sodium | 0,1 |
| agar | 13 |
| Citrate de fer ammoniacal | 0,3 |
| Glucose | 0,2 |
| TRIS | 0,1 |
| Taurocholate de sodium | 2,5 |

Puis ajout d'arbutine et/ou de CHE-beta-glucoside suivant les milieux :

| Concentration en g/l | Milieu T1 | milieu A1 | milieu A2 | milieu A3 | milieu T2 | milieu B1 | milieu B2 | milieu B3 |
|---|---|---|---|---|---|---|---|---|
| CHE-b-glucoside | 0 | 0 | 0 | 0 | 0.3 | 0.3 | 0.3 | 0.3 |
| Arbutine g/l | 0 | 0.1 | 0.3 | 1 | 0 | 0.1 | 0.3 | 1 |

Les références et les ribotypes respectifs des souches testées sont indiqués dans le tableau suivant :

| N° des souches | Ribotype |
|---|---|
| 06 03 006 | O27 |
| 08 01 092 | O27 |
| 08 01 104 | O27 |
| 08 01 109 | O27 |
| 08 01 112 | O103 |
| 08 01 113 | O27 |
| 08 01 116 | O27 |
| 08 01 118 | O27 |

### 2. ESSAI

Les milieux sont répartis en boites de Pétri.

L'ensemencement est effectué à partir de pré-cultures de 48h à 37°C en anaérobiose. Une suspension en eau physiologique à 0,5 McF est réalisée puis les souches sont ensemencées à l'oese calibrée de 10µl.

Des lectures sont effectuées après 24h d'incubation à 37°C en anaérobiose.

### 3. RESULTATS

Echelle de lecture de l'activité enzymatique/ coloration
- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

La coloration obtenue avec ce substrat est grise jusqu'à une intensité de 1 et noire pour les autres valeurs.

| | | T1 | A1 | A2 | A3 | T2 | B1 | B2 | B3 |
|---|---|---|---|---|---|---|---|---|---|
| *Clostridium difficile 109* | 24h | 0 | 0 | 0 | 0 | 1 | 1 | 4 | 4 |
| *Clostridium difficile 104* | 24h | 0 | 0 | 0 | 0 | 0,5 | 1 | 4 | 4 |
| *Clostridium difficile 092* | 24h | 0 | 0 | 0 | 0 | 0 | 1 | 4 | 4 |
| *Clostridium difficile 006 (ATCC 027)* | 24h | 0 | 0 | 0 | 0 | 1 | 1 | 4 | 4 |
| *Clostridium difficile 116* | 24h | 0 | 0 | 0 | 0 | 1 | 1 | 4 | 4 |
| *Clostridium difficile 118* | 24h | 0 | 0 | 0 | 0 | 0,5 | 1 | 3 | 4 |
| *Clostridium difficile 113* | 24h | 0 | 0 | 0 | 0 | 1 | 1 | 4 | 4 |
| *Clostridium difficile 112* | 24h | 0 | 0 | 0 | 0 | 0 | 4 | 4 | 4 |

Ainsi, après 24h d'incubation, on obtient : en nombre de souches colorées en noir sur les différents milieux :

| | T1 | A1 | A2 | A3 | T2 | B1 | B2 | B3 |
|---|---|---|---|---|---|---|---|---|
| *Intensité de Coloration >= 1* | 0/8 | 0/8 | 0/8 | 0/8 | 4/8 | **8/8** | **8/8** | **8/8** |

### 4. INTERPRETATION

En absence de substrat, aucune coloration n'est observée.

L'arbutine seule ne permet pas non plus d'obtenir une coloration des colonies.

Par contre, dès l'ajout d'arbutine en présence de CHE-beta-glucoside, on note une activation de la beta-glucosidase qui se traduit par une intensification de la coloration (grise à noire).

### 5. CONCLUSION :

L'arbutine agit comme un activateur de beta-glucosidase sur les souches de *Clostridium difficile.*

### Exemple 2: test d'une concentration optimale en arbutine sur différentes souches de C. difficile.

### 1. MILIEU ET MICROORGANISMES

Vingt quatre souches de *Clostridium difficile,* de la collection de la Demanderesse, ont été testées sur 5 milieux comprenant chacun une concentration différente en arbutine. La lecture des boites est ensuite effectuée à 24h.

Les milieux utilisés sont préparés sur la base de milieu chromID® *C*. *difficile* indiquée *supra,* pour l'exemple 1, à laquelle sont ajoutés respectivement du cefotaxime à 0,016 g/1, de la fungizone à 0,005 g/l et de la D-cyclosérine à 0,25 g/l (concentrations finales), et de l'arbutine selon le tableau suivant :

| | milieu 1 | milieu 2 | milieu 3 | milieu 4 | milieu 5 |
|---|---|---|---|---|---|
| Arbutine g/L | 0 | 0.75 | 1 | 1.25 | 1.5 |

Les références et les ribotypes respectifs des souches testées sont indiqués dans le tableau suivant :

| N° des souches | Ribotype |
|---|---|
| 02 01 042 | ND |
| 02 01 043 | ND |
| 02 06 146 | ND |
| 02 06 148 | ND |
| 06 03 006 | 027 |
| 08 01 078 | 027 |
| 08 01 080 | O27 |
| 08 01 084 | O27 |
| 08 01 088 | 010 |
| 08 01 092 | 027 |
| 08 01 094 | 078 |
| 08 01 095 | 027 |
| 08 01 096 | 027 |
| 08 01 098 | 027 |
| 08 01 100 | 027 |
| 08 01 102 | 001 |
| 08 01 103 | 014 |
| 08 01 104 | 027 |
| 08 01 109 | 027 |
| 08 01 111 | 027 |
| 08 01 113 | 027 |
| 08 01 114 | 002 |
| 08 01 116 | 027 |
| 08 01 118 | 027 |

| | |
|---|---|
| ND = non determiné | |

### 2. ESSAIS

Les milieux sont répartis en boites de Petri.

L'ensemencement est effectué à partir de pré-cultures de 48h à 37°C en anaérobiose.

Une suspension en eau physiologique à 0,5 McF est réalisée puis les souches sont ensemencées à l'oese calibrée de 10µl.

Des lectures sont effectuées après 24h d'incubation en anaérobiose.

### 3. RESULTATS

Echelle de lecture de l'activité enzymatique/ coloration
- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

La coloration obtenue avec ce substrat est grise jusqu'à une intensité de 1 et noire pour les autres valeurs.

Les résultats notés ici, sur les différents milieux, ont été obtenus après 24h d'incubation à 37°C en anaérobiose.

| | M1 | M2 | M3 | M4 | M5 |
|---|---|---|---|---|---|
| *Clostridium difficile 42* | 3 | 2,5 | 2,5 | 2,5 | 2,5 |
| *Clostridium difficile 43* | 3 | 3 | 3 | 3 | 3 |
| *Clostridium difficile 146* | 0 | 2 | 2 | 1,5 | 1,5 |
| *Clostridium difficile 148* | 3 | 3 | 3 | 3 | 3 |
| *Clostridium difficile 6* | 0,75 | 3 | 3 | 3 | 3 |
| *Clostridium difficile 78* | 3 | 3 | 3 | 3 | 3 |
| *Clostridium difficile 80* | 0,75 | 1,5 | 3 | 3 | 2,5 |
| *Clostridium difficile 84* | 2 | 2 | 2 | 2 | 2 |
| *Clostridium difficile 88* | 4 | 4 | 4 | 4 | 4 |
| *Clostridium difficile 92* | 0 | 3 | 3 | 2,75 | 2,75 |
| *Clostridium difficile 94* | 3 | 3 | 3 | 3 | 2,75 |
| *Clostridium difficile 95* | 0,5 | 3 | 3 | 2,5 | 1 |
| *Clostridium difficile 96* | 1 | 2,5 | 3 | 3 | 3 |
| *Clostridium difficile 98* | 0,75 | 3 | 3 | 3 | 3 |
| *Clostridium difficile 100* | 0,5 | 3 | 3 | 3 | 2,75 |
| *Clostridium difficile 102* | 3 | 3 | 3 | 3 | 3 |
| *Clostridium difficile 103* | 3 | 4 | 4 | 4 | 4 |
| *Clostridium difficile 104* | 0,1 | 2,5 | 3 | 3 | 3 |
| *Clostridium difficile 109* | 0,1 | 3 | 3 | 3 | 3 |
| *Clostridium difficile 111* | 0 | 2,5 | 2,5 | 2,5 | 3 |
| *Clostridium difficile 113* | 0 | 2,5 | 3 | 3 | 3 |
| *Clostridium difficile 114* | 3 | 3 | 3 | 2,5 | 2,5 |
| *Clostridium difficile 116* | 0 | 2,5 | 2,5 | 2,5 | 2,5 |
| *Clostridium difficile 118* | 0 | 2,5 | 2,5 | 2,5 | 2,5 |

Ainsi, après 24h d'incubation, on obtient, en nombre de souches colorées en noir, sur les différents milieux :

| | Milieu 1 | Milieu 2 | Milieu 3 | Milieu 4 | Milieu 5 |
|---|---|---|---|---|---|
| *Coloration >*= *1* | 12/24 dont 0/15 O27 | 24/24 dont 15/15 O27 | 24/24 dont 15/15 O27 | 24/24 dont 15/15 O27 | 24/24 dont 15/15 O27 |

### 4. INTERPRETATION

Dès l'ajout de l'arbutine dans le milieu, la coloration est nettement améliorée. A 24h, sur le milieu sans arbutine, seulement 12 souches sur 24 présentent une coloration supérieure ou égale à 1, alors qu'en présence de 750 mg/L d'arbutine, 24 souches /24 sont colorées avec une intensité minimale de 1. Les souches présentant déjà une forte coloration sur le témoin ne semblent pas ou peu impactées par la présence d'arbutine si l'on ne dépasse pas 1g/L. Au delà, on note pour certaines souches, une légère diminution de l'intensité de coloration.

### 5. CONCLUSION :

L'arbutine agit bien ici comme un activateur de beta-glucosidase et son action semble significative pour la détection précoce des *Clostridium difficile* et en particulier pour le ribotype 027.

### Exemple 3 : test de l'effet activateur de l'arbutine sur différents substrats de beta-glucosidase.

### 1. MILIEU ET MICROORGANISMES

Les huit souches de *Clostridium difficile* testées dans l'Exemple 1 ont été testées sur des milieux comprenant du DHF-beta-glucoside (milieux A), de l'alizarine- beta -glucoside (milieux B) ou du 3HF-beta -glucoside (Milieux C) et une concentration croissante en arbutine.

La lecture des boites est ensuite effectuée à 24h et 48h.

Les milieux utilisés sont préparés sur la base de milieu chromlD® *C*. *difficile* indiquée *supra,* pour l'exemple 1, à laquelle est ajoutée de l'arbutine selon le tableau suivant :

| | Milieu T | Milieu 1 | milieu 2 | milieu 3 |
|---|---|---|---|---|
| Arbutine g/L | 0 | 0.250 | 0.5 | 1 |

En fonction des substrats, les milieux sont donc les suivants :
- DHF- beta -glucoside à 400 mg/L (milieux TA, 1A, 2A et 3A)
- Alizarine- beta -glucoside à 50 mg/L (milieux TB, 1 B, 2B et 3B)
- 3HF- beta -glucoside à 300 mg/L (milieux TC, 1C, 2C et 3C)

### 2. ESSAIS

Les milieux sont répartis en boites de Petri.

L'ensemencement est effectué à partir de pré-cultures de 48h à 37°C en anaérobiose. Une suspension en eau physiologique à 0,5 McF est réalisée puis les souches sont ensemencées à l'oese calibrée de 10µl.

Des lectures sont effectuées après 24h et 48h d'incubation à 37°C et en anaérobiose.

### 3. RESULTATS

Echelle de lecture de l'activité enzymatique/ coloration
- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

Couleur des colonies :
- Milieu A :: Brun
- Milieu B :: Mauve
- Milieu C :: Brun

| | | **DHF-b-glucoside** | | | | **Alizarine-b-glucoside** | | | | **3HF-b-glucoside** | | | |
|---|---|---|---|---|---|---|---|---|---|---|---|---|---|
| | | TA | 1A | 2A | 3A | TB | 1B | 2B | 3B | TC | 1C | 2C | 3C |
| *Clostridium difficile 6* | 24h | 2 | 2,5 | 3 | 3,5 | 0,5 | 1 | 1 | 0,75 | 0 | 0 | 0 | 0 |
| | 48h | 2,5 | 2,5 | 3 | 3,5 | 0,5 | 1,5 | 1,5 | 1,5 | 0,5 | 0,5 | 1 | 1,5 |
| *Clostridium difficile 92* | 24h | 2 | 2 | 3 | 3,5 | 0,5 | 1 | 1 | 0,75 | 0 | 0 | 0 | 0 |
| | 48h | 2,5 | 2,5 | 3 | 3,5 | 0,5 | 1,5 | 1,5 | 1,5 | 0,5 | 1 | 1,5 | 1,5 |
| *Clostridium difficile 104* | 24h | 2 | 2,5 | 3 | 4 | 0,5 | 1 | 1 | 0,75 | 0,5 | 0,5 | 0,5 | 0,5 |
| | 48h | 2,5 | 2,5 | 3 | 4 | 0,5 | 1,5 | 1,5 | 1,5 | 1 | 1 | 1,5 | 2 |
| *Clostridium difficile 109* | 24h | 2 | 2,5 | 3 | 4 | 0,5 | 1 | 1 | 1 | 0 | 0 | 0 | 0 |
| | 48h | 2,5 | 3 | 3 | 4 | 0,5 | 1,5 | 1 | 1 | 1 | 1,5 | 1,5 | 1,5 |
| *Clostridium difficile 112* | 24h | 2 | 2 | 2 | 2 | 1 | 1 | 1 | 1 | 0 | 0 | 0 | 0 |
| | 48h | 3 | 3 | 3 | 2,5 | 1,5 | 1,5 | 1,5 | 1,5 | 0 | 1 | 1,5 | 1,5 |
| *Clostridium difficile 113* | 24h | 2 | 2 | 2 | 2,5 | 1 | 1 | 1 | 1 | 0 | 0 | 0 | 0 |
| | 48h | 2,5 | 2,5 | 3 | 3 | 1 | 2 | 2 | 1,5 | 1 | 1 | 1,5 | 2 |
| *Clostridium difficile 116* | 24h | 2 | 2 | 2 | 2,5 | 1 | 1,25 | 1,25 | 1 | 0,1 | 0,1 | 0,1 | 0,1 |
| | 48h | 2,5 | 2,5 | 2,5 | 3 | 1 | 1,5 | 1 | 1 | 0,5 | 1,5 | 1,5 | 1,5 |
| *Clostridium difficile 118* | 24h | 2 | 2 | 2 | 3 | 1 | 1,5 | 1,5 | 1 | 0 | 0 | 0 | 0 |
| | 48h | 2,5 | 2,5 | 3 | 3 | 1,5 | 2 | 2 | 1,5 | 0,5 | 1 | 1,5 | 1,5 |

### 4. INTERPRETATION

En présence d'arbutine, on note une amélioration de l'intensité de coloration quel que soit le substrat utilisé. Cette amélioration est surtout visible lors de l'utilisation du DHF-β-glucoside, dès 24h et essentiellement en présence de 1g/l d'arbutine. Pour l'alizarine- beta -glucoside, l'amélioration est moins nette à 24h, mais se marque davantage à 48h sur tous les milieux contenant de l'arbutine. Enfin, pour le 3HF- beta -glucoside l'apport de ce composé n'est visible qu'à 48h mais on note une réelle activation de l'activité beta -glucosidase quelle que soit la concentration utilisée et quelles que soient les souches.

Cependant, la concentration idéale varient selon le substrat utilisé et serait proche de 1g/l avec le DHF- beta -glucoside et le 3HF- beta -glucoside et de 500mg/l avec l'alizarine- beta -glucoside.

### 5. CONCLUSION :

Cet essai démontre l'intérêt de l'arbutine pour l'activation de l'activité beta -glucosidase chez les souches de *Clostridium difficile.*

### Exemple 4: test de l'effet activateur des composés Ar-beta-glucoside sur l'activité beta-glucosidase.

### 1. MILIEU ET MICROORGANISMES

Six souches de *Clostridium difficile* ont été testées sur des milieux comprenant un composé qui pourrait agir comme activateur de la beta-glucosidase chez ces souches. Les ribotypes de ces souches sont indiqués dans les tableaux *supra.* Celui de la souche référencée 08 01 091 est 056. Les composés testés comme activateurs de beta-glucosidase sont les suivants :
- Arbutine;
- O-methyl b-glucoside;
- Cellobiose ;
- Salicine ;
- 4 Aminophenyl-b-glucoside ;
- 4-méthyl-umbelliférone-b-glucoside ;
- esculine.

Ils ont été testés à des concentrations permettant d'avoir la même molarité pour chacun des composés.

Les milieux utilisés sont préparés sur la base de milieu chromlD® C. *difficile* indiquée *supra* pour l'exemple 1, à laquelle est ajouté le substrat CHE-beta-glucoside à 0,3 g/l et, respectivement, un des composés selon le tableau suivant :

| | Arbutine | O-méthyl-b-Glu | Cellobiose | Salicine | 4AminoPhe | 4MU-b-glu | esculine |
|---|---|---|---|---|---|---|---|
| **T** | - | - | - | - | - | | |
| **M1** | 1 g/l | - | - | - | - | | |
| **M2** | - | 0,7 g/l | - | - | - | | |
| **M3** | - | - | 1,25 g/l | - | - | | |
| **M4** | - | - | - | 1 g/l | - | | |
| **M5** | - | - | - | - | 1 g/l | | |
| **M6** | | | | | | 1,25 g/l | |
| **M7** | | | | | | | 1,25 g/l |

### 2. ESSAIS

Les milieux sont répartis en boites de Petri.

L'ensemencement est effectué à partir de pré-cultures de 48h à 37°C en anaérobiose.

Une suspension en eau physiologique à 0,5 McF est réalisée puis les souches sont ensemencées à l'oese calibrée de 10µl.

Des lectures sont effectuées après 24h d'incubation à 37°C et en anaérobiose.

### 3. RESULTATS

Echelle de lecture de l'activité enzymatique/ coloration
- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

La coloration obtenue avec ce substrat est grise jusqu'à une intensité de 1 et noire pour les autres valeurs.

| | T | M1 | M2 | M3 | M4 | M5 | M6 | M7 |
|---|---|---|---|---|---|---|---|---|
| *Clostridium difficile 91* | 4 | 4 | 0 | 4 | 4 | 4 | 4 | 4 |
| *Clostridium difficile 94* | 0.5 | 3 | 0 | 0,5 | 4 | 4 | 3 | 3 |
| *Clostridium difficile* 6* | 4/1 | 4 | 0 | 4/1 | 4 | 4 | 3 | 3/1 |
| *Clostridium difficile 111* | 0,5 | 4 | 0 | 0 | 4 | 3 | 3 | 3 |
| *Clostridium difficile 112* | 0 | 4 | 0 | 0 | 4 | 4 | 4 | 4 |
| *Clostridium difficile 116* | 0,5 | 3 | 0 | 0,5 | 4 | 4 | 3 | 3 |

| | | | | | | | | |
|---|---|---|---|---|---|---|---|---|
| * = souche présentant des colonies de couleurs hétérogènes ; les résultats indiqués sous forme de fraction x/y signifient que certaines souches présentent une intensité x et d'autres présentent une intensité y. | | | | | | | | |

### 4. INTERPRETATION

Le O-méthyl- beta -glucoside et le cellobiose n'ont pas d'effet activateur sur la beta -glucosidase des *Clostridium difficile.* Au contraire, on note une diminution de la coloration sur les souches présentant une coloration sur le témoin.

Par contre, comme pour l'arbutine, on observe activation de la beta -glucosidase et donc une augmentation de la coloration des colonies en présence de salicine, de 4-Aminophényl-beta-glucoside, de 4-méthyl-umbelliferone ou d'esculine. On note également une homogénéisation de la coloration pour les souches hétérogènes, en présence de l'un de ces composés. Pour le milieu contenant de l'esculine, on observe une forte diffusion de la coloration noire dans la gélose, rendant parfois difficile la lecture du milieu.

### 5. CONCLUSION :

Cet essai démontre l'effet activateur de la beta -glucosidase des *C*. *difficile* par des composés possédant au moins un cycle aromatique tels que l'arbutine, la salicine, le 4-Aminophényl-beta-glucoside, le 4-méthyl-umbelliferone ou l'esculine.

### Exemple 5 : test de 2 substrats de beta-glucosidase à base d'ALDOL™ (ALDOL™ 455-beta-glucoside et ALDOL™484-beta-glucoside - BIOSYNTH)

### 1. MILIEU ET MICROORGANISMES

Huit souches de *Clostridium difficile* ont été testées sur 4 milieux différents, respectivement un milieu témoin de croissance (T), un milieu (1) contenant 75mg/L en ALDOL™ 455-beta-glucoside, un milieu (2) contenant 75mg/L en ALDOL™ 484-beta-glucoside, et un milieu (3) contenant du CHE-b-glucoside à raison de 300mg/L et du citrate de fer ammoniacal (300mg/L).

La composition de la base nutritive commune à tous les milieux est indiquée ci-dessous. Il s'agit de la base chromlD® *C*. *difficile* exempte de citrate de fer ammoniacal mais supplémentée avec 1g/L d'arbutine.

| Composés | Conc. en g/l |
|---|---|
| Extraits tissulaires et cellulaires | 12,5 |
| Chlorure de sodium | 6 |
| Sulfate de Magnesium | 0,3 |
| L-arginine | 1 |
| Bisulfite de sodium | 0,1 |
| agar | 13 |
| Arbutine | 1 |
| Glucose | 0,2 |
| TRIS | 0,1 |
| Taurocholate de sodium | 2,5 |

Après fonte des agars, la base est séparée en 4*80mL, répartis dans les flacons T, 1, 2 et 3. Dans le flacon 3, on aura au préalable pesé 300mg/L de CHE- beta -glucoside et 300mg/L de citrate de fer ammoniacal. Un cycle d'autoclave permet de stériliser les milieux. Après retour des milieux en surfusion, les substrats à base d'ALDOL™ sont ajoutés en additifs, solubilisés dans un solvant de type DMSO (dimethylsulfoxide), à partir d'une solution-mère concentrée.

### 2. ESSAIS

Les milieux sont répartis en boites de Petri.

L'ensemencement est effectué à partir de pré-cultures de 48h à 37°C en anaérobiose.

Une suspension en eau physiologique à 0,5 McF est réalisée puis les souches sont ensemencées à l'oese calibrée de 10µl selon la méthode des 3 cadrans.

Des lectures sont effectuées après 24h d'incubation à 37°C et en anaérobiose. Les résultats sont consignés ci-après.

### 3. RESULTATS

Suite à l'hydrolyse enzymatique des divers substrats par les souches de *C. difficile,* les colorations des colonies isolées obtenues sur les milieux 1, 2 et 3 sont respectivement jaune, orange et brune.

Echelle de lecture de l'expression de l'activité enzymatique (intensités de coloration)
- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

| | | T | 1 | 2 | 3 |
|---|---|---|---|---|---|
| *Clostridium difficile 0801108* | 24h | 0 | 3 | 4 | 2 |
| *Clostridium difficile 0801105* | 24h | 0 | 3 | 3 | 4 |
| *Clostridium difficile 0801088* | 24h | 0 | 3 | 4 | 4 |
| *Clostridium difficile 0801091* | 24h | 0 | 3 | 4 | 3 |
| *Clostridium difficile 0801094* | 24h | 0 | 3 | 3 | 3 |
| *Clostridium difficile 0603006* | 24h | 0 | 2 | 3 | 2 |
| *Clostridium difficile 0801112* | 24h | 0 | 3 | 3 | 4 |
| *Clostridium difficile 0801116* | 24h | 0 | 3 | 3 | 4 |

### 4. INTERPRETATION

Les substrats ALDOL™ 455-beta-glucoside et ALDOL™484-beta-glucoside (BIOSYNTH) permettent une excellente détection des C. *difficile.* Les intensités de colorations (jaune et orange respectivement) obtenues sont élevées, et ce dès 24h d'incubation. Les performances obtenues avec ces deux substrats sont sensiblement identiques à celles obtenues avec le CHE- beta -glucoside.

## Revendications

1. Milieu réactionnel comprenant au moins un substrat de beta-glucosidase et un composé de formule Ar-beta-D-glucoside où Ar- désigne un composé aromatique, différent dudit substrat et choisi parmi l'arbutine ou hydroquinone-beta-D-glucopyranoside, le 4-methylumbelliferylbeta-glucoside, le 4-aminophenyl-beta-glucoside, la salicine ou 2-(hydroxymethyl)phenyl-beta-D-glucoside, à une concentration inférieure à 5 g/l.

2. Milieu réactionnel selon la revendication 1, dans lequel le composé Ar-beta-D-glucoside est à une concentration comprise entre 0,3 et 1,5 g/l.

3. Milieu réactionnel selon l'une des revendications 1 à 2, dans lequel le composé Ar-beta-D-glucoside est l'arbutine.

4. Milieu réactionnel selon l'une des revendications 1 à 3, dans lequel le substrat de beta-glucosidase est choisi parmi l'Alizarine-beta-glucoside, le Magenta-beta-glucoside (5-Bromo-6-chloro-3-indoxyl-beta-glucoside), le DHF-beta-glucoside, le 3HF-beta-glucoside et le CHE-beta-glucoside (3,4-Cyclohexenoescuietine-beta-glucoside) et les ALDOL™ beta-glucoside.

5. Milieu réactionnel selon l'une des revendications 1 à 4, dans lequel le substrat de beta-glucosidase est à une concentration comprise entre 25 et 1000 mg/l et plus préférentiellement entre 50 et 400 mg/l.

6. Milieu réactionnel selon l'une des revendications 1 à 5, comprenant en outre un activateur de la germination des spores de *Clostridium diffcile.*

7. Milieu réactionnel selon la revendication 6 dans lequel ledit activateur de la germination des spores de *Clostridium difficile* est le taurocholate de sodium.

8. Milieu réactionnel selon la revendication 6 ou 7, dans lequel le taurocholate de sodium est à une concentration comprise entre 0,1 et 10 g/l, préférentiellement compris entre 1 et 5 g/l.

9. Procédé de détection et/ou d'identification de *Clostridium difficile* comprenant les étapes consistant à :
a) mettre en contact un échantillon susceptible de contenir *Clostridium difficile* avec un milieu réactionnel comprenant au moins un substrat de beta-glucosidase et un composé de formule Ar-beta-D-glucoside, différent dudit substrat, où Ar- désigne un composé aromatique, choisi parmi l'arbutine ou hydroquinone-beta-D-glucopyranoside, le 4-methylumbelliferyl-beta-glucoside, le 4-aminophenyl-beta-glucoside, la salicine ou 2-(hydroxymethyl)phenyl-beta-D-glucoside, à une concentration inférieure à 5 g/l;
b) incuber en atmosphère anaérobie ;
c) détecter l'hydrolyse du substrat de beta-glucosidase, indiquant la présence de *Clostridium difficile.*

10. Procédé selon la revendication 9, dans lequel le milieu mis en oeuvre à l'étape a) est un milieu selon l'une des revendications 1 à 8.

11. Procédé selon l'une des revendications 9 ou 10 dans lequel l'incubation mise en oeuvre à l'étape b) est réalisée en anaérobiose.

12. Utilisation de l'arbutine en tant qu'activateur de beta-glucosidase dans un milieu de détection et/ou d'identification de *Clostridium difficile.*

## Patentansprüche

1. Reaktionsmedium, umfassend mindestens ein Substrat von beta-Glucosidase und eine Verbindung der Formel Ar-beta-D-glucosid, wobei Ar- für eine von dem Substrat verschiedene aromatische Verbindung steht und aus Arbutin oder Hydrochinon-beta-D-glucopyranosid, 4-Methylumbelliferyl-beta-glucosid, 4-Aminophenyl-beta-glucosid, Salicin oder 2-(Hydroxymethyl)phenyl-beta-D-glucosid ausgewählt ist, in einer Konzentration von weniger als 5 g/l.

2. Reaktionsmedium nach Anspruch 1, wobei die Verbindung Ar-beta-D-glucosid in einer Konzentration zwischen 0,3 und 1,5 g/l vorliegt.

3. Reaktionsmedium nach einem der Ansprüche 1 bis 2, wobei es sich bei der Verbindung Ar-beta-D-glucosid um Arbutin handelt.

4. Reaktionsmedium nach einem der Ansprüche 1 bis 3, wobei das Substrat von beta-Glucosidase aus Alizarin-beta-glucosid, Magenta-beta-glucosid (5-Brom-6-chlor-3-indoxyl-beta-glucosid), DHF-beta-glucosid, 3HF-beta-glucosid und CHE-beta-glucosid (3,4-Cyclohexenoesculetin-beta-glucosid) und den ALDOL™-beta-glucosiden ausgewählt ist.

5. Reaktionsmedium nach einem der Ansprüche 1 bis 4, wobei das Substrat von beta-Glucosidase in einer Konzentration zwischen 25 und 1000 mg/l und stärker bevorzugt zwischen 50 und 400 mg/l vorliegt.

6. Reaktionsmedium nach einem der Ansprüche 1 bis 5, das außerdem einen Aktivator der Keimung der Sporen von *Clostridium difficile* umfasst.

7. Reaktionsmedium nach Anspruch 6, wobei es sich bei dem Aktivator der Keimung der Sporen von *Clostridium difficile* um Natriumtaurocholat handelt.

8. Reaktionsmedium nach Anspruch 6 oder 7, wobei das Natriumtaurocholat in einer Konzentration zwischen 0,1 und 10 g/1, vorzugsweise zwischen 1 und 5 g/l, vorliegt.

9. Verfahren zum Nachweis und/oder zur Identifikation von *Clostridium difficile,* wobei man in den folgenden Schritten:
a) eine Probe, die möglicherweise *Clostridium difficile* enthält, mit einem Reaktionsmedium in Kontakt bringt, umfassend mindestens ein Substrat von beta-Glucosidase und eine von dem Substrat verschiedene Verbindung der Formel Ar-beta-D-glucosid, wobei Ar- für eine aromatische Verbindung steht, die aus Arbutin oder Hydrochinon-beta-D-glucopyranosid, 4-Methylumbelliferyl-beta-glucosid, 4-Aminophenyl-beta-glucosid, Salicin oder 2-(Hydroxymethyl)phenyl-beta-D-glucosid ausgewählt ist, in einer Konzentration von weniger als 5 g/l;
b) unter einer anaeroben Atmosphäre inkubiert;
c) die Hydrolyse des Substrats von beta-Glucosidase nachweist, die auf das Vorhandensein von *Clostridium difficile* hindeutet.

10. Verfahren nach Anspruch 9, wobei es sich bei dem in Schritt a) verwendeten Medium um ein Medium nach einem der Ansprüche 1 bis 8 handelt.

11. Verfahren nach einem der Ansprüche 9 oder 10, wobei die in Schritt b) durchgeführte Inkubation unter Anaerobiose vorgenommen wird.

12. Verwendung von Arbutin als Aktivator von beta-Glucosidase in einem Medium zum Nachweis und/oder zur Identifikation von *Clostridium difficile.*

## Claims

1. A reaction medium comprising at least one beta-glucosidase substrate and a compound of the formula Ar-beta-D-glucoside where Ar- designates an aromatic compound, different from said substrate and selected from amongst arbutine or hydroquinone-beta-D-glucopyranoside, 4-methylumbelliferyl-beta-glucoside, 4-aminophenyl-beta-glucoside, salicin or 2-(hydroxymethyl)phenyl-beta-D-glucoside, aesculin or 6,7-dihydroxy-coumarin-beta-glucoside, at a concentration lower than 5 g/l.

2. The reaction medium according to claim 1, wherein the compound Ar-beta-D-glucoside is at a concentration of between 0.3 and 1.5 g/l.

3. The reaction medium according to claim 1 or 2, wherein the compound Ar-beta-D-glucoside is arbutine.

4. The reaction medium according to one of claims 1 to 3, wherein the beta-glucosidase substrate is selected from amongst Alizarin-beta-glucoside, Magenta-beta-glucoside (5-Bromo-6-chloro-3-indoxyl-beta-glucoside), DHF-beta-glucoside, 3HF-beta-glucoside and CHE-beta-glucoside (3,4-Cyclohexenoesculetine-beta-glucoside) and ALDOL™ beta-glucoside.

5. The reaction medium according to one of claims 1 to 4, wherein the beta-glucosidase substrate is at a concentration of between 25 and 1000 mg/l, and more preferably between 50 and 400 mg/l.

6. The reaction medium according to one of claims 1 to 5, further comprising a *Clostridium difficile* spore germination activator.

7. The reaction medium according to claim 6, wherein said *Clostridium difficile* spore germination activator is sodium taurocholate.

8. The reaction medium according to claim 7 or 8, wherein sodium taurocholate is at a concentration of between 0.1 and 10 g/l, preferably of between 1 and 5 g/l.

9. A process of detecting and/or identifying *Clostridium difficile* comprising the steps consisting of:
a) placing a sample likely to contain *Clostridium difficile* in contact with a reaction medium comprising at least one beta-glucosidase substrate and a compound of the formula Ar-beta-D-glucoside, different from said substrate, where Ar- designates an aromatic compound, selected from amongst arbutine or hydroquinone-beta-D-glucopyranoside, 4-methylumbeiliferyl-beta-glucoside, 4-aminophenyl-beta-glucoside, salicin or 2-(hydroxymethyl)phenyl-beta-D-glucoside, aesculin or 6,7-dihydroxy-coumarin-beta-glucoside, at a concentration lower than 5 g/l.
b) incubating in anaerobiosis;
c) detecting the hydrolysis of the beta-glucosidase substrate, which indicates the presence of *Clostridium difficile.*

10. A process according to claim 9, wherein the medium employed in step a) is a medium according to one of claims 1 to 8.

11. The process according to one of claims 9 or 10 wherein the incubation employed in step b) is performed in anaerobiosis.

12. Use of arbutin as a beta-glucosidase activator in a *Clostridium difficile* detection and/or identification medium.
